(19) Europäisches Patentamt

European Patent Office

Office européen des brevets

(11) **EP 0 595 666 B1**

(12) **FASCICULE DE BREVET EUROPEEN**

(45) Date de publication et mention
de la délivrance du brevet:
**01.12.1999 Bulletin 1999/48**

(51) Int. Cl.⁶: **A61B 8/06**, G01P 5/00, G01F 1/66

(21) Numéro de dépôt: **93402283.1**

(22) Date de dépôt: **20.09.1993**

(54) **Sonde et procédé pour déterminer avec précision la vitesse ou le débit d'un milieu liquide**

Sonde und Verfahren zur genauen Bestimmung der Geschwindigkeit oder des Durchflusses einer Flüssigkeit

Probe and procedure for a precise determination of velocity or flow of a liquid

(84) Etats contractants désignés:
**AT BE CH DE DK ES FR GB GR IE IT LI LU NL PT SE**

(30) Priorité: **21.09.1992 FR 9211425
24.05.1993 US 64900**

(43) Date de publication de la demande:
**04.05.1994 Bulletin 1994/18**

(60) Demande divisionnaire:
**97104304.7 / 0 787 977**

(73) Titulaire:
**INSTITUT NATIONAL DE LA SANTE ET DE LA RECHERCHE MEDICALE (INSERM)
75654 Paris Cédex 13 (FR)**

(72) Inventeurs:
• **Cathignol, Dominique
F-69740 Genas (FR)**
• **Lavandier,,Bernard
F-42820 Ambierle (FR)**
• **Muchada, Raoul
F-69007 Lyon (FR)**

(74) Mandataire: **Portal, Gérard et al
Cabinet Beau de Loménie
158, rue de l'Université
75340 Paris Cédex 07 (FR)**

(56) Documents cités:
EP-A- 0 035 325        EP-A- 0 363 156
DE-B- 2 509 568        FR-A- 2 296 165
FR-A- 2 424 733        US-A- 4 757 822

• IEEE TRANSACTIONS ON BIO-MEDICAL ENGINEERING, Mars 1974, NEW YORK US pages 168 - 171 R.OLSON ET J.COOKE 'A Nondestructive Ultrasonic Technique to Measure Diameter and Blood Flow in Arteries'
• ISA TRANSACTIONS, vol.18, no.1, 1979, PITTSBURGH US pages 57 - 61 M.WELLS ET AL. 'Ultrasonic transesophageal measurement of Hemodynamic Parameters in Humans'
• ULTRASONICS, vol.17, no.5, Septembre 1979, GUILDFORD GB pages 215 - 218 M.HISTAND ET AL. 'Ultrasonic pulsed Doppler transoesophageal measurement of aortic haemodynamics in humans'
• MEDICAL AND BIOLOGICAL ENGINEERING, vol.14, no.2, Mars 1976, STEVENHAGE,GB pages 245 - 246 A.SAINZ 'Vessel area detection with c.w. ultrasound'
• IEEE TRANSACTIONS ON BIO-MEDICAL ENGINEERING, vol.27, no.10, Octobre 1980, N.Y.,US pages 565 - 573 E.WILDI 'Dynamics and Limitations of Blood/Muscle Interface...'

**Description**

La présente invention concerne le domaine technique des sondes ultrasonores, au sens général, utilisées pour procéder à des mesures de vitesse et/ou de débit et elle vise en particulier le domaine des sondes ultrasonores aptes à assurer des mesures intracorporelles, en étant introduites dans le corps humain par un orifice naturel.

L'invention trouve une application particulièrement avantageuse pour la mesure du débit aortique par l'intermédiaire de la sonde introduite à l'intérieur de l'oesophage.

Le document EP-0363156, Cardiometrics, décrit un appareil et un procédé pour mesurer le débit du sang avec un cathéter introduit dans le conduit sanguin, dans lequel un transducteur à faisceau large et un transducteur à faisceau étroit sont montés. Le faisceau large se trouve dans un plan longitudinal passant par l'axe longitudinal de sorte que son faisceau ne peut couvrir toute la section du vaisseau.

Le document FR-A-2 424 733 a proposé une sonde intracorporelle composée d'un cathéter formant une gaine souple contenant un flexible relié, par l'une de ses extrémités, à un bloc-support sur lequel est monté au moins un transducteur ultrasonore connecté, extérieurement au cathéter, à une unité de commande et de traitement. L'autre extrémité du flexible est montée solidaire d'un organe d'entraînement en rotation du bloc-support sur lui-même.

Une telle sonde permet, par effet Doppler, de déterminer la vitesse et, par suite, si le diamètre est par ailleurs connu, le débit du sang circulant à l'intérieur d'un vaisseau. Il est rappelé que le débit est égal à la section du vaisseau multiplié par la vitesse spatiale moyenne à l'intérieur du vaisseau.

Si la sonde décrite ci-dessus a permis d'effectuer un progrès important en ce qui concerne la mesure des débits aortiques, il apparaît que les mesures de débit effectuées par une telle sonde manquent de précision.

En effet, une telle sonde assure la mesure de la vitesse moyenne qui est effectuée à l'aide d'un transducteur ultrasonore dont le faisceau ne couvre pas la totalité de la surface de la section droite du vaisseau. De plus, en pratique, il apparaît difficile, voire impossible, de déterminer la bonne orientation de la sonde, afin de procéder aux mesures de débits. En effet, des positions incorrectes de la sonde peuvent donner lieu à des signaux Doppler ayant toutes les apparences du signal recherché mais conduisant à des valeurs inexactes du débit. Il s'ensuit que le faisceau ultrasonore peut couvrir aussi partiellement une zone située au dehors du vaisseau, de sorte que le dispositif de mesure prend en compte des éléments mobiles extérieurs au vaisseau, ce qui est une source d'erreur.

Il est à noter également que, dans le dispositif antérieur, le traitement effectué sur le signal Doppler ne permet pas d'exclure le mouvement des parois du vaisseau, puisque tous les signaux provenant des cibles mobiles situées tout au long du faisceau ultrasonore sont pris en compte.

Par ailleurs, au cours du cycle cardiaque, et en particulier pendant la diastole, le profil de vitesse spatiale fait apparaître des zones de vitesses nulles ou suffisamment faibles pour ne pas pouvoir être mesurées à l'aide de la sonde et du dispositif de traitement utilisé conjointement. La vitesse moyenne spatiale ainsi mesurée est erronée, puisque seules les cibles animées d'un mouvement suffisamment rapide sont prises en compte. Dans ces conditions, la mesure du débit conduit à une erreur extrêmement importante dans la mesure où la vitesse moyenne mesurée est alors multipliée par la section totale du vaisseau. Cette erreur est d'autant plus importante que la surface couverte par les veines liquidiennes immobiles ou suffisamment lentes pour ne pas être détectées par la sonde est elle-même importante par rapport à la section du vaisseau.

La présente invention vise donc à remédier aux inconvénients ci-dessus en proposant un procédé et une sonde capables d'améliorer notablement la précision de la mesure de la vitesse spatiale d'une cible mobile pour une mesure précise du débit de la cible.

Un autre objet de l'invention vise à proposer un procédé et une sonde aptes à assurer une mesure précise de vitesse exclusivement sur toute la section de la cible mobile.

Un autre objet de l'invention vise à proposer un procédé et une sonde capables d'effectuer des mesures précises de vitesse et, par suite, de débit, dans un fluide comprenant des particules en suspension, circulant dans un conduit, cette circulation pouvant être en particulier de type pulsé et ainsi présenter à certains instants une vitesse nulle ou même un flux rétrograde.

Un autre objet de l'invention vise à offrir une sonde capables d'effectuer des mesures précises de vitesse et, par suite, de débit, du sang dans l'aorte. Avantageusement, la solution doit permettre de mesurer la vitesse et donc le débit du sang dans l'aorte par l'emploi d'une sonde de type endocavitaire introduite dans l'oesophage jusqu'à être positionnée en regard de l'aorte, aussi bien pendant la diastole que la systole, ou des débits présentant des veines liquidiennes de vitesse variable notamment en raison d'un débit irrégulier ou pulsé, ce qui peut inclure une vitesse nulle ou même un flux rétrograde.

Pour atteindre les objectifs ci-dessus, la sonde comporte au moins un bloc-support sur lequel est monté au moins un transducteur ultrasonore connecté à une unité de commande et de traitement.

Selon un premier aspect de l'invention, on fournit une sonde permettant de mesurer la vitesse ou le débit d'un fluide contenant des particules en suspension entraînées par ledit fluide en circulation dans un premier conduit ayant un axe longitudinal, pendant que ladite sonde est placée dans un deuxième conduit extérieur au premier conduit, la sonde comprenant :

a) une gaine ayant également un axe longitudinal, et un bloc-support;

b) au moins un transducteur ultrasonore à faisceau étroit connecté à une unité de commande et de traitement, monté sur ledit bloc-support pourvu de moyens de rotation pour réaliser une rotation dudit transducteur à faisceau étroit autour de l'axe longitudinal de la gaine indépendamment de la gaine pour orienter ledit transducteur à faisceau étroit avec son faisceau étroit coupant ledit premier conduit selon un plan de coupe sensiblement perpendieulaire à l'axe longitudinal dudit premier conduit et donnant une section totale du premier conduit dans lequel le fluide circule;

c) au moins un transducteur ultrasonore à faisceau large, fixé sur ledit bloc-support pour présenter son faisceau large dans une position relative connue par rapport à celle dudit transducteur ultrasonore à faisceau étroit, les moyens de rotation étant également prévus pour faire varier en rotation le transducteur ultrasonore à faisceau large autour de l'axe longitudinal de la gaine indépendamment de la gaine pour mesurer, par effet Doppler, la vitesse du fluide circulant dans ledit premier conduit, le transducteur à faisceau large présentant un faisceau capable de couvrir la totalité de la surface de la section droite du premier conduit dans lequel le fluide circule; et

d) ladite unité de commande et de traitement comprenant des moyens de mesure et de traitement des caractéristiques du signal du transducteur à faisceau étroit, pour déterminer l'orientation perpendiculaire du transducteur à faisceau étroit par rapport au premier conduit, ladite unité de commande commandant le fonctionnement du transducteur à faisceau large pour mesurer la vitesse du fluide s'écoulant dans ledit premier conduit quand le transducteur à faisceau étroit atteint ladite orientation, permettant ainsi une mesure de la vitesse de l'ensemble des veines de fluide circulant dans ledit premier conduit.

Selon une variante de réalisation, le transducteur à faisceau étroit et le transducteur à faisceau large présentent un plan de symétrie commun.

Selon encore une autre variante de réalisation, le transducteur à faisceau large et le transducteur à faisceau étroit sont montés sur un bloc support unique, et orientés l'un relativement à l'autre de manière que leur faisceau ou l'axe moyen du faisceau se trouve décalé d'un angle de divergence θ, le transducteur à faisceau large étant orientable par rapport au conduit de manière à présenter une inclinaison relative θ par rapport à l'axe longitudinal du conduit, pour assurer l'effet Doppler, tandis que le transducteur à faisceau étroit est dans cette position orientable, selon une direction sensiblement perpendiculaire à l'axe longitudinal du conduit et coupant ledit conduit, afin de déterminer la position relative précise du bloc-support des transducteurs relativement au conduit.

Selon encore une autre variante de réalisation, les moyens de mesure et de traitement du signal du transducteur à faisceau étroit permettent de déterminer la position proximale ou la position distale ou encore à la fois les positions distales et proximales des bords du conduit dans le plan de coupe réalisé par ledit transducteur à faisceau étroit.

Selon encore une autre variante de réalisation, les moyens de mesure et de traitement du signal du transducteur à faisceau étroit comportent des moyens pour déterminer l'amplitude des signaux reçus par le transducteur et des moyens pour détecter les maximums d'amplitude de ces signaux qui correspondent respectivement au bord proximal ou au bord distal ou encore à la fois aux bords proximal et distal dudit conduit dans ledit plan de coupe.

Selon encore une autre variante de réalisation, l'unité de commande et de traitement comporte des moyens de calcul pour déterminer la plage de distance $(P_2-P_1)$ correspondant au bord proximal et au bord distal du plan de coupe du conduit réalisé par le transducteur à faisceau large, à partir de la connaissance de l'angle de divergence (θ) précité ainsi que de la distance entre le bord proximal et le bord distal du conduit dans le plan de coupe du transducteur à faisceau étroit, et des moyens de sélection des signaux reçus par le transducteur à faisceau large, dans la plage de distance $(P_2-P_1)$ déterminée par les moyens de calcul.

Selon encore une autre variante de réalisation, la sonde est caractérisée en ce qu'elle comprend des moyens pour faire varier en rotation le(s) transducteur(s) précité(s) relativement au conduit, de manière à déterminer l'orientation recherchée pour laquelle le transducteur à faisceau étroit est orienté sensiblement perpendiculairement à l'axe longitudinal du conduit et coupant ledit conduit.

Selon encore une autre caractéristique de la sonde précitée, celle-ci est caractérisée en ce que l'unité de commande et de traitement comporte des moyens de traitement des signaux issus du transducteur à faisceau étroit pour déterminer l'orientation du transducteur à faisceau large pour laquelle le nombre de particules en suspension dans le volume défini comme intersection du faisceau large et du conduit soit maximal.

Selon un mode de réalisation particulier, l'unité de commande et de traitement comporte également des moyens aptes à mesurer la vitesse et/ou l'énergie rétrodiffusée par les particules en suspension dans le fluide circulant dans ledit conduit, pilotés par

- des moyens aptes à déterminer des instants précis de prise en compte de la vitesse et/ou de l'énergie rétrodiffusée mesurée(s), l'un de ces instants correspondant à une énergie rétrodiffusée maximale du fait qu'à cet instant un maximum de particules sont détectées comme en mouvement dans le volume de mesure du transducteur à faisceau large, et d'autres instants correspondant à des mesures d'énergie rétrodiffusée instantanée ;

- et des moyens de correction de la vitesse ou du débit par un facteur dépendant de l'énergie rétrodiffusée à chaque instant et de l'énergie rétrodiffusée maximale correspondant à la phase de circulation où l'ensemble des particules en suspension contenues dans une section droite sont en mouvement.

Selon encore une autre variante de réalisation, l'unité de commande et de traitement comporte des moyens de comparaison des énergies rétrodiffusées respectivement maximale et instantanée avec un seuil donné (N), à partir duquel la correction de vitesse ou de débit est effectuée.

En particulier, le seuil donné (N) est compris entre 10 et 50 % de l'énergie rétrodiffusée maximale (E$_S$) et, de préférence, est de l'ordre de 25 %.

La sonde selon l'invention peut selon un autre mode de réalisation, être caractérisée en ce qu'elle comprend un cathéter formant une gaine souple contenant un flexible relié par l'une de ses extrémités au bloc-support des transducteurs connectés, extérieurement au cathéter, à l'unité de commande et de traitement, l'autre extrémité du flexible étant reliée à un moyen d'orientation ou de mise en rotation permettant de réaliser l'orientation/rotation recherchée individuellement des transducteurs relativement à l'axe longitudinal de la sonde.

Selon encore une autre variante, la sonde est de type endocavitaire et est introduite dans l'oesophage jusqu'à être positionnée en regard de l'aorte.

Selon l'invention, la sonde permet de réaliser des mesures exclusivement sur la section totale d'un milieu ou d'un conduit donné, tel que l'aorte, grâce au fait que le transducteur ultrasonore à faisceau large projette un faisceau large ultrasonore couvrant la section complète du conduit.

Selon un deuxième aspect, l'invention comprend un procédé tel que défini dans la revendication 14.

Diverses autres caractéristiques ressortent des revendications et de la description faite ci-dessous en référence aux dessins annexés qui montrent, à titre d'exemples non limitatifs, des formes de réalisation et de mise en oeuvre de l'objet de l'invention.

La figure 1 est une coupe en élévation d'un mode de réalisation actuellement préféré de l'invention, représenté schématiquement en position opératoire dans l'oesophage en regard de l'aorte.

La figure 2 est une vue en projection de la coupe transversale prise sensiblement selon les lignes II-II de la figure 1 et montrant un détail caractéristique de l'invention.

La figure 3 est un diagramme illustrant, dans le temps, l'énergie rétrodiffusée par la cible mobile, à savoir ici le sang circulant dans l'aorte.

La figure 4 est un exemple d'une courbe de vitesse en fonction du temps, obtenue à partir de la sonde selon l'invention selon les figures 1 et 2 avant correction (schématisé en traits discontinus) et après correction.

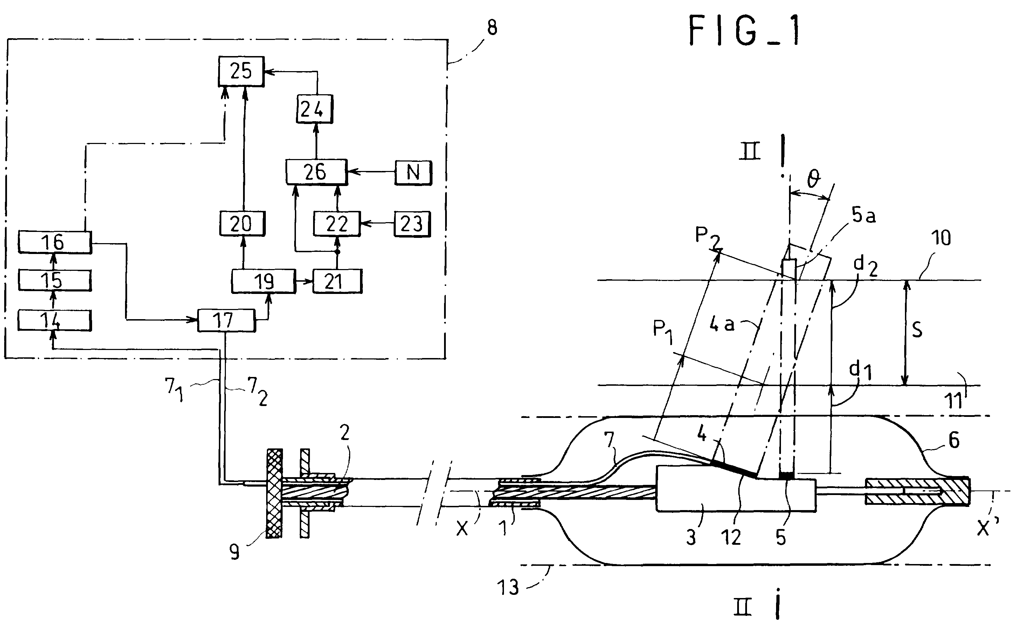

La figure 1 montre un exemple de réalisation d'une sonde conforme à l'invention, destinée à assurer des mesures intracorporelles de vitesse et/ou de débit. A cet effet, la sonde intracorporelle comprend un cathéter formant une gaine ou un tube souple réalisé de manière connue par des matières choisies pour leurs caractéristiques de non-toxicité et de bonne tolérance pour les muqueuses. La gaine 1 contient un flexible 2 qui est relié, par l'une de ses extrémités, à au moins un bloc-support 3 sur lequel sont montés des transducteurs ultrasonores 4 et 5. D'une manière classique, cette extrémité de la sonde, qui est destinée à être introduite dans le corps, est munie d'un ballonnet 6 entourant le bloc-support 3. Les transducteurs 4, 5 sont reliés à un câble électrique 7 placé dans la gaine 1 et sortant à l'extérieur du cathéter pour être connecté à une unité 8 de commande des transducteurs et de traitement des signaux délivrés par ces derniers. L'extrémité du flexible 2, opposée de celle pourvue du bloc-support 3, est reliée à un organe de manoeuvre 9, tel qu'un bouton moleté assurant une rotation sur lui-même du flexible 2.

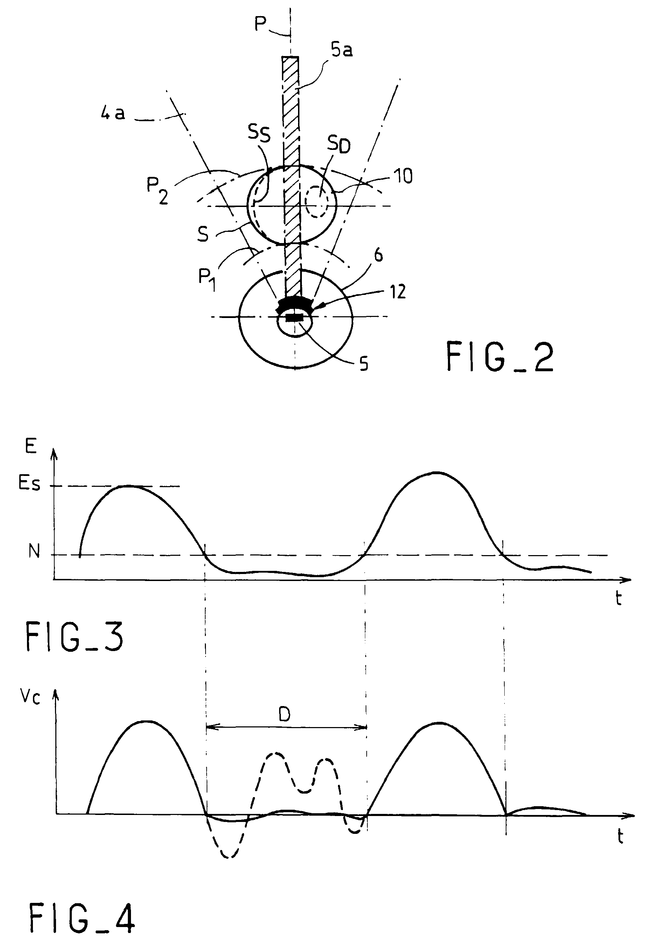

Selon une caractéristique de l'invention, le bloc-support 3 est aménagé pour recevoir au moins un transducteur ultrasonore 4, présentant un faisceau dit large 4a, à savoir adapté pour couvrir au moins toute la section S d'un milieu 10, tel qu'un vaisseau formant, de préférence, l'aorte et occupé par une cible mobile 11 formée, par exemple, par un flux sanguin (figure 2). Le transducteur 4 est placé sur un plan d'appui 12 ménagé dans le bloc support 3. Dans le cas où la sonde est destinée à être introduite dans l'oesophage pour permettre la mesure du débit aortique, le transducteur 4 est incliné par rapport à l'axe longitudinal x-x' commun au flexible 2 et au bloc-support 3, de manière à permettre une détection de vitesse du sang par effet Doppler. Par exemple, le transducteur 4 utilisé est le transducteur du type nature P1 60, de forme en secteur cylindrique de dimensions 4/4 mm, de rayon de courbure de 6 mm et commercialisé par la Société Quartz et Silice.

Selon l'invention, le bloc-support 3 est équipé, également, d'un transducteur ultrasonore 5 présentant un faisceau 5a considéré étroit par rapport à la section S de l'aorte 10 et par rapport au faisceau 4a. Le transducteur 5, du type à faisceau plan ou localisé, est monté sur le bloc-support 3, de manière que son faisceau 5a se trouve, de préférence, centré sur le plan symétrique P du faisceau large, passant par l'axe x-x'. Le faisceau étroit 5a est décalé d'un angle de divergence θ par rapport au faisceau large 4a et se trouve, de préférence, orienté selon une direction sensiblement perpendiculaire a l'axe x-x',

Bien entendu, il doit être considéré que le transducteur 5 peut être fixé sur le bloc-support 3, de telle manière que son faisceau 5a se trouve dans une position relative différente mais connue par rapport à celle du faisceau large 4a. De plus, les transducteurs 4 et 5 peuvent être montés sur des blocs-supports distincts dont leurs positions relatives sont connues, en particulier par construction.

La sonde décrite dans l'exemple illustré est destinée à être introduite par un orifice naturel dans une voie naturelle, telle que l'oesophage 13 représenté en traits mixtes, puis déplacée axialement afin que les transducteurs 4 et 5 se trouvent placés en regard d'une section S de l'aorte 10. Le bloc-support 3 est alors déplacé sur lui-même par l'intermédiaire du bouton de manoeuvre 9 transmettant son effet par le flexible 2, de manière à orienter convenablement les transducteurs 4, 5 en azimut.

Afin de placer le faisceau large 4a dans une position où il couvre la section du vaisseau 10 à explorer, le transducteur 5 à faisceau étroit est relié à l'unité 8 qui comporte des moyens de mesure et de traitement des caractéristiques du signal du transducteur à faisceau étroit 5.

A cet effet, l'unité de commande et de traitement 8 comporte des moyens 14 reliés au transducteur 5 par une liaison $7_1$ et conçus pour déterminer l'amplitude des signaux reçus en écho par le transducteur à faisceau étroit 5. Les moyens de détermination 14 sont reliés à des moyens 15 conçus pour détecter les maximums de l'amplitude des signaux réfléchis. D'une manière classique, il est à considérer que l'amplitude des échos du transducteur 5 est maximum lorsque son faisceau 5a est perpendiculaire aux parois du vaisseau 10. Le bloc-support 3 est déplacé en azimut par l'intermédiaire du bouton 9, jusqu'à ce qu'il occupe une position dans laquelle l'amplitude des échos du signal du transducteur 5 apparaisse maximum. Le transducteur 4 se trouve alors, par construction, convenablement orienté, de sorte que son faisceau large 4a insonifie toute la section S de l'aorte 10. Il est ainsi possible de donner une seule et unique orientation correcte à la sonde, dans la mesure où l'amplitude des échos des parois décroît de façon importante pour un faible écart angulaire. Par ailleurs, il est à noter que le transducteur ultrasonore 4 permet de mesurer la vitesse sur toute la section du vaisseau, dans la mesure où toute cette section est insonifiée par le faisceau large 4a.

Il est à noter que, dans le cas où deux transducteurs 5 à faisceau étroit sont utilisés, il peut être envisagé de détecter, par exemple, la position dans laquelle l'amplitude des signaux des deux transducteurs 5 est égale, afin de déterminer l'orientation convenable du transducteur à faisceau large 4.

Afin d'augmenter encore la précision des mesures de vitesses par le transducteur 4, il est prévu de prendre en compte uniquement les échos du transducteur 5 provenant d'un intervalle $P_2$-$P_1$ qui circonscrit la section de l'aorte 10 en deux points extrêmes opposés. A cet effet, l'unité de commande et de traitement 8 comprend des moyens 16 conçus pour déterminer cet intervalle $P_2$-$P_1$. Les moyens 16 sont reliés aux moyens 15, afin de déterminer l'intervalle $d_2$-$d_1$ correspondant aux deux points extrêmes de l'aorte détectés à partir des maximums de l'amplitude des échos relatifs aux signaux du transducteur 5. La connaissance de l'intervalle $d_2$-$d_1$ permet de calculer la section puisqu'il est connu ou considéré que le vaisseau est de section circulaire.

Les moyens 16 déterminent, ensuite, l'intervalle $P_2$-$P_1$ à partir de l'intervalle $d_2$-$d_1$ et de l'angle de divergence θ entre les deux faisceaux, connu en particulier par construction. Ces moyens de détermination 16 pilotent des moyens de sélection 17 reliés au transducteur 4 par une liaison $7_2$. Ces moyens 17 permettent de sélectionner uniquement les échos des signaux du transducteur 4 qui sont obtenus dans une plage de temps de réponse correspondant à l'intervalle $P_2$-$P_1$. Les moyens de sélection 17 sont reliés à des moyens de traitement classique 19 assurant l'obtention d'un signal Doppler. Ces moyens de traitement 19 sont connectés à des moyens 20 connus en soi, aptes à déterminer la vitesse moyenne spatiale $V_m$ du sang traversant la section S de l'aorte 10.

L'utilisation combinée d'au moins un transducteur à faisceau large 4 et d'au moins un transducteur à faisceau étroit 5 autorise la mesure des vitesses sur toute la section de l'aorte 10 sans prendre en compte d'éléments situés à l'extérieur d'un tel vaisseau. Le champ de mesure de vitesse coïncide, ainsi, de la façon la plus étroite possible avec la section S de l'aorte.

Selon une caractéristique avantageuse de l'invention, la sonde est apte à assurer des mesures précises de vitesse moyenne spatiale qui tiennent compte de la section des veines liquidiennes animées d'une vitesse nulle ou suffisamment faible pour être considérée comme nulle par les moyens 19, 20 fonctionnant de façon classique. La sonde selon l'invention est ainsi apte à assurer des mesures de vitesse qui tiennent compte de la section effective ou réelle occupée par les hématies considérées en mouvement à l'intérieur de l'aorte.

A cet effet, l'unité 8 comprend des moyens 21 aptes à mesurer l'énergie rétrodiffusée par les particules en mouvement, à savoir les hématies dans le cas du sang. L'énergie rétrodiffusée E, qui est proportionnelle au nombre d'hématies en mouvement, est mesurée à chaque instant, afin de connaître la masse de liquide en mouvement (figure 3). Ainsi, l'énergie E du signal reçu est donnée par la formule suivante :

$$E = e.c.l.S.$$

avec c étant la concentration en particules, à savoir en hématies, e l'énergie rétrodiffusée par une partieule et le produit I.S. étant le volume de mesure dans lequel se trouvent les particules en mouvement.

Les moyens de calcul de l'énergie 21, qui reçoivent le signal Doppler issu des moyens de traitement 19, déterminent à chaque instant l'amplitude ou l'énergie E rétrodiffusée par les cibles en mouvement. L'amplitude de signal Doppler est proportionnelle à la racine carrée de l'énergie rétrodiffusée. La sortie des moyens de calcul 21 est connectée à des moyens 22 conçus pour laisser passer l'énergie rétrodiffusée à un ou plusieurs instants définis, en particulier lors de la systole. Les moyens 22 sont ainsi reliés à des moyens 23 aptes à déterminer lesdits instants définis et en particulier l'instant où se produit la systole. D'une manière classique, la systole peut être détectée à partir de la vitesse maximale du sang, de l'énergie rétrodiffusée ou d'un électrocardiogramme.

Les moyens 22 délivrent donc la valeur de l'énergie rétrodiffusée $E_S$ pendant la systole. De préférence, l'énergie rétrodiffusée $E_S$, lors de la systole, est mesurée sur plusieurs cycles cardiaques, par exemple de l'ordre d'une dizaine, puis moyennée, afin de tenir compte des variations physiologiques normales.

Il doit être considéré que pendant la systole toutes les hématies sont en mouvement, de sorte que l'énergie totale rétrodiffusée $E_S$ à cet instant correspond au mouvement des cibles occupant la section totale S du vaisseau. En dehors de la systole et, notamment pendant la diastole, la surface $S_D$ couverte par les particules effectivement en mouvement est susceptible de se trouver réduite par rapport à la section complète S.

La prise en compte de l'énergie rétrodiffusée, lors de la systole $E_S$ et lors de la diastole $E_D$, permet de déterminer la surface réelle ou effective théorique $S_D$ participant au débit. Une telle surface est telle que :

$$S_D = S.(E_D/E_S) = S.K.$$

Le facteur de correction K est déterminé par des moyens de correction 24 reliés aux moyens 21, 22. D'une manière avantageuse, les moyens de correction 24 pondèrent le facteur K par un coefficient de correction pratique qui tient compte des caractéristiques techniques du transducteur 4 utilisé et des moyens 19, en particulier de la valeur minimale des vitesses détectées et de la bande passante du signal Doppler. Ces moyens de correction 24 sont connectés à des moyens 25 qui sont reliés aux moyens 20 de détermination de la vitesse moyenne spatiale. Ces moyens 25 permettent de calculer, à partir des valeurs de la vitesse moyenne spatiale et du facteur de correction K, la vitesse moyenne $V_C$ corrigée et, par suite, le débit du sang en mouvement sur la surface localisée $S_D$, à partir de la connaissance de la section du vaisseau.

La figure 4 illustre un exemple de courbe donnant la vitesse corrigée $V_C$ en fonction du temps. Cette courbe permet d'apprécier la correction effectuée à partir des vitesses brutes de mesure qui sont schématisées en traits discontinus lors de la diastole D. Le procédé selon l'invention permet d'obtenir une précision élevée sur les mesures de vitesses et, par suite, de débits du sang, puisque ces valeurs mesurées tiennent compte de la section effective participant au débit de sang.

Tel que cela apparaît plus précisément à la figure 3, le facteur de correction déterminé par les moyens 24 est appliqué uniquement lorsque l'énergie rétrodiffusée est inférieure à un seuil N donné, de nature fixe ou réglable, pour tenir compte à la fois des variations physiologiques et des variations statistiques normales du signal Doppler connues par ailleurs. Avantageusement, le seuil M est compris entre 10 et 50% de l'énergie rétrodiffusée maximale $E_S$ pendant la systole et, de préférence, de l'ordre de 25%. Une telle comparaison est effectuée par des moyens 26 interposés entre les moyens 21-22 et 24 permet de corriger à chaque instant du cycle cardiaque les valeurs de vitesse et de débit, particulièrement lors de la diastole D, comme cela apparait plus précisément à la figure 4.

Bien entendu, les divers moyens constitutifs de l'unité 8 peuvent être réalisés d'une manière programmée ou câblée. De plus, il est à noter que les différents circuits nécessaires au fonctionnement des transducteurs 4, 5 n'ont pas été décrits plus précisément, car ils ne font pas partie de l'invention et sont connus en soi. Par ailleurs, il doit être considéré que la description qui précède concerne une sonde intracorporelle ou endocavitaire. Bien entendu, il est clair que l'objet de l'invention peut être appliqué à une sonde extracorporelle. Dans ce cas, la sonde ne comporte pas le cathéter 1 et le flexible 2. Par exemple, il peut être envisagé d'utiliser une telle sonde afin de mesurer le débit de l'aorte ascendante par voie susternale.

Dans les dessins, la section $(S_S)$ est définie par la section occupée par les particules en suspension dans le conduit, par exemple les hématies dans l'aorte, lorsque la totalité ou sensiblement la totalité est considérée en mouvement, dans le plan de balayage de la coupe du transducteur à faisceau large 4, ladite section $(S_S)$ étant sensiblement égale à la section (S) du conduit balayé, ici l'aorte, comme représenté à la figure 2. La section partielle $(S_D)$ est la section occupée à chaque instant par les particules en suspension dans le conduit, par exemple les hématies détectées comme étant en mouvement et qui peuvent représenter une zone très localisée comme montré en figure 2.

Par rapport au facteur de correction (K), le facteur de correction (K) peut dans un exemple de réalisation être représenté par la formule suivante :

$$K = \left(\frac{E_D}{E_S}\right)^n \times k$$

dans laquelle :

K = facteur de correction

$E_D$ = énergie partielle rétrodiffusée comme précédemment définie

$E_S$ = énergie totale rétrodiffusée comme précédemment définie

n = est un nombre constituant un autre facteur de correction

k = est un coefficient de correction comme précédemment défini dépendant des caractéristiques techniques du transducteur (4) et des moyens émettant, recevant, mesurant et traitant les signaux liés au transducteur (4) à effet Doppler, incluant les moyens de mesure (19).

Lors de la mesure de la vitesse du flux de sang dans l'aorte, les valeurs suivantes ont été obtenues sur deux patients :

- avec la sonde selon l'invention, le coefficient de correction k est égal à 1, n est égal à ½ et le seuil (N) est fixé à 25 % de l'énergie maximale rétrodiffusée ($E_S$).

Pour ces deux patients, les signaux reçus par les transducteurs de la sonde selon l'invention sont enregistrés et soumis à des calculs par ordinateur et ont donné les valeurs suivantes de mesure de vitesse :

A - Pour le patient A, ayant un diamètre d'aorte de 3 cm :

- vitesse moyenne sur 5OO mesures de vitesse instantanée, non corrigée : 8,64 cm/seconde

Sur la courbe enregistrée, qui a été réalisée pendant 1O secondes, il a été observé que les mesures concernant la section partielle ($S_D$) étaient en majorité négatives.

Puisque ce seuil était applicable, une correction de la vitesse moyenne a été réalisée en fournissant une valeur de vitesse moyenne corrigée de 9,3 cm/seconde.

Un second essai a été réalisé ultérieurement sur le même patient A et les valeurs étaient comme suit :

- vitesse moyenne sur 5OO mesures de vitesse instantanée, pendant 1O secondes, non corrigée : 9,66 cm/seconde.

Il a été observé qu'en majorité, les flux localisés ($S_D$) étaient positifs et en outre que le seuil était applicable.

La vitesse moyenne corrigée était en conséquence de 9,O3 cm/seconde.

B - Pour un second patient B, ayant un diamètre d'aorte de 2,4 cm, dans les mêmes conditions, les valeurs de vitesse étaient comme suit :

- vitesse moyenne, non corrigée : 18,6 cm/seconde.

Avec le patient B, les flux localisés ($S_D$) étaient en majorité positifs et le seuil était également applicable.

La valeur de vitesse corrigée était de 15,36 cm/seconde.

L'invention n'est pas limitée aux exemples décrits et représentés, car diverses modifications peuvent y être apportées sans sortir de son cadre.

## Revendications

1. Sonde permettant de mesurer la vitesse ou le débit d'un fluide contenant des particules en suspension entraînées par ledit fluide en circulation dans un premier conduit (10) ayant un axe longitudinal pendant que ladite sonde est placée dans un deuxième conduit (13) extérieur au premier conduit (10), la sonde comprenant :

a) une gaine (1) ayant également un axe longitudinal, et un bloc-support (3);

b) au moins un transducteur ultrasonore (5) à faisceau étroit (5a) connecté à une unité de commande et de traitement (8), monté sur ledit bloc-support (3) pourvu de moyens de rotation (9) pour réaliser une rotation dudit transducteur (5) à faisceau étroit autour de l'axe longitudinal de la gaine (1) indépendamment de la gaine (1) pour orienter ledit transducteur à faisceau étroit (5) avec son faisceau étroit (5a) coupant ledit premier conduit (10) selon un plan de coupe sensiblement perpendiculaire à l'axe longitudinal dudit premier conduit (10) et donnant une section totale du premier conduit (10) dans lequel le fluide circule;

c) au moins un transducteur ultrasonore (4) à faisceau large (4a), fixé sur ledit bloc-support (3) pour présenter son faisceau large dans une position relative connue par rapport à celle dudit transducteur ultrasonore (5) à faisceau étroit (5a), les moyens de rotation (9) étant également prévus pour faire varier en rotation le transducteur ultrasonore (4) à faisceau large (4a) autour de l'axe longitudinal de la gaine (1) indépendamment de la gaine (1) pour mesurer, par effet Doppler, la vitesse du fluide circulant dans ledit premier conduit (10), le transducteur (4) à faisceau large (4a) présentant un faisceau capable de couvrir la totalité de la surface de la section droite du premier conduit (10) dans lequel le fluide circule; et

d) ladite unité de commande et de traitement (8) comprenant des moyens (14,15) de mesure et de traitement des caractéristiques du signal du transducteur (5) à faisceau étroit (5a), pour déterminer l'orientation perpendiculaire du transducteur (5) à faisceau étroit par rapport au premier conduit (10), ladite unité de commande (8) commandant le fonctionnement du transducteur à faisceau large (4) pour mesurer la vitesse du fluide s'écoulant dans ledit premier conduit (10) quand le transducteur (5) à faisceau étroit (5a) atteint ladite orientation, permettant ainsi une mesure de la vitesse de l'ensemble des veines de fluide circulant dans ledit premier conduit (10).

2. Sonde selon la revendication 1, dans laquelle le transducteur (5) à faisceau étroit et le transducteur (4) à faisceau large (4a) présentent un plan de symétrie commun.

3. Sonde selon la revendication 1 ou 2, dans laquelle le transducteur (5) à faisceau étroit et le transducteur (4) à faisceau large sont orientés l'un relativement à l'autre de manière que leur faisceau ou l'axe moyen du faisceau se trouve décalé d'un angle de divergence ($\theta$), de sorte que lorsque le transducteur (5) à faisceau étroit (5a) est orienté selon une direction sensiblement perpendiculaire à l'axe longitudinal du premier conduit (10) afin de déterminer la position relative précise du bloc-support des transducteurs relativement au premier conduit, le transducteur (4) à faisceau large (4a) est orienté par rapport au premier conduit de manière à présenter une inclinaison relative par rapport à l'axe longitudinal du premier conduit, pour assurer l'effet Doppler.

4. Sonde selon l'une des revendications Précédentes, dans laquelle les moyens de mesure et de traitement du signal du transducteur (5) à faisceau étroit (5a) permettent de déterminer la position proximale ou la position distale ou encore à la fois les positions distale et proximale des bords du premier conduit dans le plan de coupe réalisé par ledit transducteur (5) à faisceau étroit (5a).

5. Sonde selon la revendication 4, dans laquelle les moyens de mesure et de traitement du signal du transducteur (5) à faisceau étroit (5a) comportent des moyens (14) pour déterminer l'amplitude des signaux reçus par le transducteur (5) et des moyens (15) pour détecter les maximums d'amplitude de ces signaux qui correspondent respectivement au bord proximal ou au bord distal ou encore à la fois aux bords proximal et distal dudit premier conduit (10) dans ledit plan de coupe.

6. Sonde selon la revendication 4 ou 5, dans laquelle l'unité de commande et de traitement (8) comporte des moyens de calcul (16) pour déterminer la plage de distances correspondant au bord proximal et au bord distal du plan de coupe du conduit réalisé par le transducteur (4) à faisceau large (4a), à partir de la connaissance de l'angle de divergence (8) précité ainsi que de la distance entre le bord proximal et le bord distal du conduit dans le plan de coupe du transducteur (5) à faisceau étroit (5a), et des moyens (17) de sélection des signaux reçus par le transducteur (4) à faisceau large (4a), dans la plage de distances déterminée par les moyens de calcul (16).

7. Sonde selon l'une des revendications précédentes, dans laquelle l'unité de commande et de traitement (8) comporte des moyens de traitement (15) des signaux issus du transducteur (5) à faisceau étroit (5a) pour déterminer l'orientation du transducteur (4) à faisceau large (4a) pour laquelle le nombre de particules en suspension dans le volume défini comme intersection du faisceau large et du conduit soit maximal.

8. Sonde selon l'une des revendications précédentes, dans laquelle l'unité de commande et de traitement (8) comporte également des moyens (19,20,21) aptes à mesurer la vitesse et/ou l'énergie rétrodiffusée par les particules en suspension dans le fluide circulant dans ledit premier conduit (10), pilotés par

- des moyens (23) aptes à déterminer des instants précis de prise en compte de la vitesse et/ou de l'énergie rétrodiffusée mesurée(s), l'un de ces instants correspondant à une énergie rétrodiffusée maximale du fait qu'à cet instant un maximum de particules sont détectées comme étant en mouvement dans le volume de mesure du transducteur (4) à faisceau large (4a), et d'autres instants correspondant à des mesures d'énergie rétrodiffusée instantanée;
- et des moyens (24) de correction de la vitesse ou du débit par un facteur dépendant de l'énergie rétrodiffusée à chaque instant et de l'énergie rétrodiffusée maximale correspondant à la phase de circulation ou l'ensemble des particules en suspension contenues dans une section droite sont en mouvement.

9. Sonde selon la revendication 8, dans laquelle l'unité de commande et de traitement (8) comporte des moyens (26) de comparaison des énergies rétrodiffusées respectivement maximale et instantanée avec un seuil donné (N), à partir duquel la correction de vitesse ou de débit est effectuée.

10. Sonde selon la revendication 9, dans laquelle le seuil donné (N) est compris entre 10 et 50% de l'énergie rétrodiffusée maximale ($E_S$) et, de préférence, est de l'ordre de 25 %.

**11.** Sonde selon l'une des revendications précédentes, comprenant un cathéter (1) formant ladite gaine souple contenant un flexible (2) relié par l'une de ses extrémités au bloc-support (3) des transducteurs (4,5) et connecté, extérieurement au cathéter, à l'unité de commande et de traitement (8), l'autre extrémité du flexible étant reliée au moyen de rotation (9) permettant de réaliser l'orientation/rotation recherchée individuellement ou simultanément des transducteurs relativement à l'axe longitudinal de la sonde.

**12.** Sonde selon l'une des revendications précédentes, dans laquelle le premier conduit dans lequel le fluide circule est un vaisseau sanguin (10), et ladite sonde est adaptée pour être introduite dans un conduit naturel (13) extérieur audit vaisseau sanguin (10) et pour mesurer la vitesse ou le débit du sang s'écoulant dans ledit vaisseau sanguin (10).

**13.** Sonde selon la revendication 12 dans laquelle ledit conduit extérieur est l'oesophage (13) lorsque le vaisseau sanguin est l'aorte (10).

**14.** Procédé à l'exclusion d'un procédé de traitement thérapeutique ou d'un procédé de diagnostic à l'aide d'une sonde selon l'une des revendications 1 à 10, pour mesurer la vitesse ou le débit d'un fluide circulant dans un premier conduit (10) ayant une paroi et un axe longitudinal, comprenant les étapes de :

a) positionner ladite sonde dans un deuxième conduit (13) qui est extérieur au premier conduit (10) et extérieur au corps humain ou animal;
b) activer le transducteur (5) à faisceau étroit (5a) pour émettre un faisceau étroit (5a) ultrasonore à travers ledit premier conduit (10) traversant complètement;
c) activer les moyens de rotation (9) pour faire varier la rotation du transducteur (5) à faisceau étroit (5a), autour de l'axe longitudinal de la sonde, sensiblement parallèle à l'axe longitudinal du premier conduit (10), jusqu'à une position pour laquelle l'amplitude de l'énergie ultrasonore réfléchie indique la perpendicularité du faisceau étroit ultrasonore vis-à-vis de la paroi dudit premier conduit (10); et
d) mesurer la vitesse ou le débit à travers le premier conduit (10) par l'activation du transducteur (4) à faisceau large (4a) projetant le faisceau large (4a) ultrasonore passant au travers de la section complète du premier conduit (10), pour permettre une mesure de la vitesse de l'ensemble des veines de fluide circulant dans ledit premier conduit (10).

**Claims**

**1.** A probe for measuring the speed or flow rate of a fluid containing particles in suspension and entrained by said fluid flowing along a first duct (10) having a longitudinal axis, said probe being placed in a second duct (13) outside the first duct (10), the probe comprising:

a) a sheath (1) also having a longitudinal axis, and a support block (3);
b) at least one narrow beam ultrasound transducer (5) connected to a control and processing unit (8), the transducer being mounted on said support block (3) which is provided with rotation means (9) to cause said narrow beam transducer (5) to rotate about the longitudinal axis of the sheath (1) independently of the sheath (1) in order to orient said narrow beam transducer (5) so that its narrow beam (5a) intersects said first duct (10) in a plane of intersection substantially perpendicular to the longitudinal axis of said first duct (10) and giving a total section of the first duct (10) in which the fluid flows;
c) at least one wide beam ultrasound transducer (4) fixed on said support block (3) to present its broad beam (4a) in a known position relative to the position of said narrow beam ultrasound transducer (5), the rotation means also being designed to rotate the broad beam ultrasound transducer (4) about the longitudinal axis of the sheath (1) independently of the sheath (1) so as to measure the speed of the fluid flowing in said first duct (10) by the Doppler effect, the broad beam transducer (4) presenting a beam capable of covering the entire area of the right section of the first duct (10) in which the fluid flows; and
d) said control and processing unit (8) having means (14, 15) for measuring and processing characteristics of the narrow beam transducer signal (5) to determine the perpendicular orientation of the narrow beam transducer (5) relative to the first duct (10), said control unit (8) controlling the operation of the broad beam transducer (4) to measure the speed of the fluid flowing in said first duct (10) when the narrow beam transducer (5) reaches said orientation, thus enabling the speed of all of the fluid streams flowing in said first duct (10) to be measured.

**2.** A probe according to claim 1, in which the narrow beam transducer (5) and the wide beam transducer (4) share a common plane of symmetry.

**3.** A probe according to claim 1 or 2, in which the narrow beam transducer (5) and the wide beam transducer (4) are oriented relative to each other in such a manner that their beams or the mean axes of their beams are offset from each other at an angle of divergence ($\theta$) such that the narrow beam transducer (5) is oriented in a direction that is substantially perpendicular to the longitudinal axis of the first duct (10) so as to determine precisely the position of the transducer support block relative to the first duct, the wide beam transducer (4) is oriented relative to the first duct in such a manner as to present an inclination relative to the longitudinal axis of the first duct so as to ensure that the Doppler effect occurs.

4. A probe according to any preceding claim, in which the means for measuring and processing the signal from the narrow beam transducer (5) enable the proximal or distal position or both the distal and proximal positions of the edges of the first duct to be determined in the section plane defined by said narrow beam transducer (5).

5. A probe according to claim 4, in which the means for measuring and processing the signal from the narrow beam transducer (5) include means (14) for determining the amplitude of the signals received by the transducer (5) and means (15) for detecting amplitude maxima in said signals, which maxima correspond respectively to the proximal edge or to the distal edge or indeed to both the proximal edge and the distal edge of said first duct (10) in said second plane.

6. A probe according to claim 4 or 5, in which the control and processing unit (8) includes calculation means (16) for determining the range of distances corresponding to the proximal edge and to the distal edge of the section plane of the duct as implemented by the wide beam transducer (4) on the basis of knowledge of the above-specified angle of divergence ($\theta$) and of the distance between the proximal edge and the distal edge of the duct in the section plane of the narrow beam transducer (5), and means (17) for selecting the signals received by the wide beam transducer (4) from the range of distances determined by the calculation means (16).

7. A probe according to any preceding claim, in which the control and processing unit (8) has means (15) for processing the signals from the narrow beam transducer (5) to determine the orientation of the wide beam transducer (4) for which the number of particles in suspension in the volume defined as the intersection between the wide beam (4a) and the duct is at a maximum.

8. A probe according to any preceding claim, in which the control and processing unit (8) also includes means (19, 20, 21) suitable for measuring the speed and/or the energy backscattered by the particles in suspension in the fluid flowing in said first duct (10), said means being controlled by:

• means (23) suitable for determining precise instants at which the measured backscattered energy and/or speed is/are taken into account, one of said instants corresponding to a backscattered energy maximum because at said instant a maximum number of particles are detected as being in motion in the measurement volume of the wide beam transducer (4), and other instants corresponding to instantaneous measurements of backscattered energy; and

• means (24) for correcting the speed or the flow rate by a factor depending both on the energy backscattered at each instant and on the maximum backacattered energy corresponding to the flow stage in which all of the particles in suspension in a right section are in motion.

9. A probe according to claim 8, in which the control and processing unit (8) includes means (26) for comparing the maximum and instantaneous backscattered energies with a given threshold (N), on the basis of which said speed or flow rate correction is performed.

10. A probe according to claim 9, in which the given threshold lies in the range 10% to 50% of the maximum backscattered energy ($E_S$) and is preferably about 25%.

11. A probe according to any preceding claim, comprising a catheter (1) forming said flexible sheath containing a cable (2) connected at one of its ends to the transducer support block (3) and connected, outside the catheter, to the control and processing unit (8), the other end of the cable being connected to the rotation means (9) enabling the desired orientation and/or rotation of the transducers (4, 5) relative to the longitudinal axis of the probe to be performed individually or simultaneously.

12. A probe according to any preceding claim, in which the first duct in which the fluid flows is a blood vessel (10) and said probe is adapted to be inserted in a natural duct (13) outside said blood vessel (10) and for measuring the speed or the flow rate of blood flowing in said blood vessel (10).

13. A probe according to claim 12, in which said external duct is the esophagus (13) when the blood vessel is the aorta (10).

**14.** A method not including a method of therapeutic treatment or a method of diagnosis using a probe according to any of claims 1 to 10 to measure the speed or the flow rate of a fluid flowing in a first duct (10) having a wall and a longitudinal axis, the method comprising the steps of:

a) positioning said probe in a second duct (13) outside the first duct (10) and outside the human or animal body;
b) activating the narrow beam transducer (5) to emit a narrow ultrasound beam (5a) through said first duct (10) so as to pass right through it;
c) activating the rotation means (9) to vary the rotation of the narrow beam transducer (5) about the longitudinal axis of the probe, substantially parallel to the longitudinal axis of the first duct (10), to find a position in which the amplitude of the reflected ultrasound energy indicates that the narrow ultrasound beam is perpendicular relative to the wall of said first duct (10); and
d) measuring the speed or flow rate along the first duct (10) by activating the wide beam transducer (4) so as to send the wide ultrasound beam (4a) through the full section of the first duct (10) so as to enable the speed of all of the streams of fluid traveling in said first duct (10) to be measured.

**Patentansprüche**

**1.** Sonde zur Messung der Geschwindigkeit oder der Durchflussmenge einer Flüssigkeit enthaltend Partikel in Suspension, die durch die in einer ersten Leitung (10) mit einer Längsachse zirkulierende Flüssigkeit mitgeführt werden, während die Sonde in einer zweiten Leitung (13) außerhalb der ersten Leitung (10) vorgesehen ist, wobei die Sonde aufweist:

a) eine Hülse (1), die ebenfalls eine Längsachse und einen Trägerblock (3) aufweist;
b) zumindest einen Ultraschall-Wandler (5) mit schmalem Strahlenbündel (5a), der mit einer Steuer- und Verarbeitungseinheit (8) verbunden und auf dem Trägerblock (3) angebracht ist, der mit Rotationsmitteln (9) versehen ist, um eine Rotation des Wandlers (5) mit schmalem Strahlenbündel um die Längsachse der Hülse (1) unabhängig von der Hülse (1) zu realisieren, um den Wandler (5) mit schmalem Strahlenbündel mit seinem schmalem Strahlenbündel (5a) auszurichten, um die erste Leitung (10) gemäß einer zur Längsachse der ersten Leitung (10) im Wesentlichen rechtwinkeligen Schnittebene zu schneiden und einen Gesamtquerschnitt der ersten Leitung (10) zu erhalten, in der die Flüssigkeit zirkuliert;
c) zumindest einen Ultraschall-Wandler (4) mit breitem Strahlenbündel (4a), der zum Richten seines breiten Strahlenbündels in einer bekannten Relativposition in Bezug auf jene des Ultraschall-Wandlers (5) mit schmalem Strahlenbündel (5a) auf dem Trägerblock (3) befestigt ist, wobei die Rotationsmittel (9) auch vorgesehen sind, um den Ultraschall-Wandler (4) mit breitem Strahlenbündel (4a) um die Längsachse der Hülse (1) unabhängig von der Hülse (1) rotatorisch zu variieren, um durch den Dopplereffekt die Geschwindigkeit der in der ersten Leitung (10) zirkulierenden Flüssigkeit zu messen, wobei der Wandler (4) mit breitem Strahlenbündel (4a) ein Strahlenbündel aufweist, das die gesamte Fläche des geraden Querschnitts der ersten Leitung (10), in der die Flüssigkeit zirkuliert, bedecken kann;
d) wobei die Steuer- und Verarbeitungseinheit (8) Mittel (14, 15) zur Messung und Verarbeitung der Eigenschaften des Signals des Wandlers (5) mit schmalem Strahlenbündel (5a) aufweist, um die rechtwinkelige Ausrichtung des Wandlers (5) mit schmalem Strahlenbündel relativ zur ersten Leitung (10) zu bestimmen, wobei die Steuereinheit (8) den Betrieb des Wandlers mit breitem Strahlenbündel (4) zur Messung der Geschwindigkeit der Flüssigkeit, die in der ersten Leitung (10) fließt, steuert, wenn der Wandler (5) mit schmalem Strahlenbündel (5a) die Ausrichtung erreicht, so dass dadurch die Messung der Geschwindigkeit der Gesamtheit des in der ersten Leitung (10) zirkulierenden Flüssigkeitsstroms gewährleistet ist.

**2.** Sonde nach Anspruch 1, in der der Wandler (5) mit schmalem Strahlenbündel und der Wandler (4) mit breitem Strahlenbündel (4a) eine gemeinsame Symmetrieebene aufweisen.

**3.** Sonde nach Anspruch 1 oder 2, in der der Wandler (5) mit schmalem Strahlenbündel und der Wandler (4) mit breitem Strahlenbündel relativ zueinander so ausgerichtet sind, dass ihr Strahlenbündel oder die Mittelachse des Strahlenbündels um einen Divergenzwinkel ($\theta$) verschoben ist, so dass der Wandler (5) mit schmalem Strahlenbündel (5a) gemäß einer zur Längsachse der ersten Leitung (10) im Wesentlichen rechtwinkeligen Richtung orientiert ist, so dass die genaue Relativposition des Trägerblocks der Wandler in Bezug auf die erste Leitung bestimmt wird, wobei der Wandler (4) mit breitem Strahlenbündel (4a) in Bezug auf die erste Leitung so orientiert ist, dass er eine Neigung in Bezug auf die Längsachse der ersten Leitung für den Dopplereffekt aufweist.

4. Sonde nach einem der vorhergehenden Ansprüche, in der die Mittel für die Messung und Verarbeitung des Signals des Wandlers (5) mit schmalem Strahlenbündel (5a) es erlauben, die proximale Position oder die distale Position oder auch gleichzeitig die distale und proximale Position der Ränder der ersten Leitung in einer durch den Wandler (5) mit schmalem Strahlenbündel (5a) vorgesehenen Schnittebene zu bestimmen.

5. Sonde nach Anspruch 4, in der die Mittel für die Messung und Verarbeitung des Signals des Wandlers (5) mit schmalem Strahlenbündel (5a) Mittel (14) zur Bestimmung der Amplitude der vom Wandler (5) erhaltenen Signale und Mittel (15) zur Detektion der Maxima der Amplitude dieser Signale aufweisen, die jeweils dem proximalen Rand oder dem distalen Rand oder auch gleichzeitig dem proximalen und dem distalen Rand der ersten Leitung (10) in der Schnittebene entsprechen.

6. Sonde nach Anspruch 4 oder 5, in der die Steuer- und Verarbeitungseinheit (8) Rechenmittel (16) zur Bestimmung des Bereichs der Abstände entsprechend dem proximalen Rand und dem distalen Rand der durch den Wandler (4) mit breitem Strahlenbündel (4a) realisierten Schnittebene der Leitung, ausgehend von der Kenntnis des vorgenannten Divergenzwinkels (8) sowie des Abstandes zwischen dem proximalen Rand und dem distalen Rand der Leitung in der Schnittebene des Wandlers (5) mit schmalem Strahlenbündel (5a), und Mittel (17) zur Wahl der vom Wandler (4) mit breitem Strahlenbündel (4a) erhaltenen Signale in dem Bereich der Abstände, der durch die Rechenmittel (16) bestimmt ist, aufweist.

7. Sonde nach einem der vorhergehenden Ansprüche, in der die Steuer- und Verarbeitungseinheit Mittel (15) zur Verarbeitung der vom Wandler (5) mit schmalem Strahlenbündel (5a) abgegebenen Signale aufweist, um die Orientierung des Wandlers (4) mit breitem Strahlenbündel (4a) zu bestimmen, für die die Anzahl der Partikel in Suspension in dem als Schnitt des breiten Strahlenbündels und der Leitung definierten Volumen maximal ist.

8. Sonde nach einem der vorhergehenden Ansprüche, in der die Steuer- und Verarbeitungseinheit (8) weiters Mittel (19, 20, 21) aufweist, die zur Messung der Geschwindigkeit und/oder der durch die Partikel in Suspension in der in der ersten Leitung (10) zirkulierenden Flüssigkeit rückgestrahlten Energie geeignet sind, welche gesteuert sind durch

- Mittel (23), geeignet zur Bestimmung der exakten Momente der Registrierung der Geschwindigkeit und/oder der gemessenen rückgestrahlten Energie, wobei einer dieser Momente einer maximalen rückgestrahlten Energie entspricht, da zu diesem Moment ein Maximum an Partikeln als in dem Messvolumen des Wandlers (4) mit breitem Strahlenbündel (4a) in Bewegung befindlich detektiert wird, und wobei andere Momente Messungen von momentan rückgestrahlter Energie entsprechen;

- und Mittel (24) zur Korrektur der Geschwindigkeit oder der Durchflussmenge um einen Faktor, der von der rückgestrahlten Energie zu jedem Moment und von der maximalen rückgestrahlten Energie abhängt, die der Phase der Zirkulation entspricht, wo die Gesamtheit der in einem geraden Querschnitt enthaltenen Partikel in Suspension in Bewegung ist.

9. Sonde nach Anspruch 8, in der die Steuer- und Verarbeitungseinheit (8) Mittel (26) zum Vergleich der jeweils maximalen und momentanen rückgestrahlten Energie mit einem gegebenen Schwellwert (N), von dem ausgehend die Korrektur der Geschwindigkeit oder der Durchflussmenge durchgeführt wird, aufweist.

10. Sonde nach Anspruch 9, in der der gegebene Schwellwert (N) zwischen 10 und 50 % der maximalen rückgestrahlten Energie $(E_S)$ liegt und vorzugsweise in der Größenordnung von 25 % liegt.

11. Sonde nach einem der vorhergehenden Ansprüche, mit einem Katheter (1), der die weiche Hülse formt, welche ein flexibles Stück (2) aufweist, das an einem seiner Enden mit dem Trägerblock (3) der Wandler (4, 5) verbunden ist und außerhalb des Katheters mit der Steuer- und Verarbeitungseinheit (8) verbunden ist, wobei das andere Ende des flexiblen Stücks mit dem Rotationsmittel (9) verbunden ist, um die gewünschte individuelle oder gleichzeitige Orientierung/Rotation der Wandler relativ zur Längsachse der Sonde zu erlauben.

12. Sonde nach einem der vorhergehenden Ansprüche, in der die erste Leitung, in der die Flüssigkeit zirkuliert, ein Blutgefäß (10) ist und die Sonde geeignet ist, um in eine natürliche Leitung (13) außerhalb des Blutgefäßes (10) zur Messung der Geschwindigkeit oder Durchflussmenge des in dem Blutgefäß (10) fließenden Bluts eingeführt zu werden.

13. Sonde nach Anspruch 12, in der die äußere Leitung die Speiseröhre (13) ist, während das Blutgefäß die Aorta (10) ist.

14. Verfahren, ausgenommen ein Verfahren zur therapeutischen Behandlung oder ein Diagnoseverfahren, mit Hilfe einer Sonde nach einem der Ansprüche 1 bis 10, zur Messung der Geschwindigkeit oder der Durchflussmenge einer zirkulierenden Flüssigkeit in einer ersten Leitung (10) mit einer Wand und einer Längsachse, mit den Schritten:

a) Positionieren der Sonde in einer zweiten Leitung (13), die sich außerhalb der ersten Leitung (10) und außerhalb des menschlichen oder tierischen Körpers befindet;

b) Aktivieren des Wandlers (5) mit schmalem Strahlenbündel (5a) zum Abgeben eines schmalen Ultraschall-Strahlenbündels (5a) quer durch die erste Leitung (10), wobei es diese vollständig durchquert;

c) Aktivieren der Rotationsmittel (9) zum Variieren der Rotation des Wandlers (5) mit schmalem Strahlenbündel (5a) um die Längsachse der Sonde, die im Wesentlichen parallel zur Längsachse der ersten Leitung (10) ist, bis in eine Position, bei der die Amplitude der rückgestrahlten Ultraschallenergie die senkrechte Richtung des schmalen Ultraschall-Strahlenbündels gegenüber der Wand der ersten Leitung (10) anzeigt; und

d) Messen der Geschwindigkeit oder der Durchflussmenge quer durch die erste Leitung (10) durch Aktivieren des Wandlers (4) mit breitem Strahlenbündel (4a), der das breite Ultraschall-Strahlenbündel (4a) aussendet, dass es durch den kompletten Querschnitt der ersten Leitung (10) durchgeht, um eine Messung der Geschwindigkeit der Gesamtheit des in der ersten Leitung (10) zirkulierenden Flüssigkeitsstroms zu ermöglichen.

FIG_1

FIG_2

FIG_3

FIG_4